**Europäisches Patentamt**

**European Patent Office**

**Office européen des brevets**

(19)

(11) **EP 1 216 725 A2**

(12) # EUROPEAN PATENT APPLICATION

(43) Date of publication:
26.06.2002 Bulletin 2002/26

(51) Int Cl.7: **A61N 2/02**

(21) Application number: 01310502.8

(22) Date of filing: 17.12.2001

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **18.12.2000 JP 2000404337**

(71) Applicant: **Hakuju Institute for Health Science
Co., Ltd
Tokyo (JP)**

(72) Inventors:
• **Hara, A., c/o Hakuju Inst. for Health Sc. Co.,Ltd.
Tokyo (JP)**
• **Uenaka, N., Hakuju Inst. for Health Sc. Co.,Ltd.
Tokyo (JP)**

(74) Representative: **Read, Matthew Charles et al
Venner Shipley & Co.
20 Little Britain
London EC1A 7DH (GB)**

(54) **Electric potential therapy apparatus and control method of optimal dose amount for human body area**

(57) The present invention relates to an electric potential therapy apparatus for applying a high voltage to respective areas of a human body for treatment and control method of an optimal dose amount for a human body area. An electric potential therapy apparatus comprises an electric potential treatment device provided with a main electrode and an opposed electrode; a high voltage generation apparatus for applying a high voltage to these respective electrodes; induced current control means for causing an extremely small amount of induced current to flow in respective areas composing a human body trunk with control of the body surface electric field by varying the applied voltage to be applied to the main electrode and opposed electrode and the distance between the opposed electrode and the human body trunk surface; and a power source for driving the high voltage generation apparatus. A control method of an optimal dose amount for a human body area comprises the steps of: applying a high voltage to the electrode; controlling a dose amount of a product of an induced current value flowing in areas composing a human body trunk and an induced current flowing time; and supplying the dose amount to respective areas of a human body trunk.

Fig. 1

**Description**

BACKGROUND OF THE INVENTION

1. Field of the Invention

The present invention relates to an electric potential therapy apparatus for performing a treatment by applying a high voltage to respective areas of a human body and a control method of optimal dose amount for respective areas of a human body.

2. Description of Related Art

In general, an electric potential therapy apparatus is designed to be suitable for a commercial AC power source of 100 volt (V):50/60 Hertz (Hz), 120 V:50/60 Hz, and 200 or 220 V:50/60 Hz. Such a conventional electric potential therapy apparatus generates naturally an electric field near the human body surface, by applying a high voltage. And the electric field presents an electric field intensity in the vicinity of the surface of respective areas of an uneven human body. However, conventionally, the electric potential treatment has been performed observing the whole body only macroscopically, and a fine control of the electric field intensity has not been performed for respective areas of the body surface. In short, in the conventional electric potential therapy apparatus, a main electrode and an opposed electrode have been installed to perform the electric potential treatment by placing the human body between these electrodes. Therefore, it is impossible to obtain a sufficient electric potential treatment effect.

SUMMARY OF THE INVENTION

The present invention has been achieved in view of the problems mentioned above, and it is therefore an object of the invention to obtain an electric potential therapy apparatus for performing an electric potential treatment by flowing very little induced current in respective areas of a human body trunk, controlling the electric field intensity, in respect of trunk areas of an uneven human body. Also, it is another object to provide a method for controlling an optimal dose amount for respective areas of a human body (affected part, among others), namely optimal application supply amount thereof (it is obtained by a product of an induced current value flowing in respective areas composing a human body trunk and time for flowing the induced current, or the product of applied voltage, sum of voltages of one electrode and another electrode, and applying time).

An electric potential therapy apparatus comprises an electric potential treatment device provided with a main electrode and an opposed electrode, a high voltage generation apparatus for applying a high voltage to the respective electrodes, induced current control means for causing an extremely small amount of induced current to flow in respective areas composing a human body trunk with control of an electric field of the body trunk, by varying the applied voltage to be applied to the respective electrodes and the distance between the opposed electrode and the trunk surface of the human body, and a power source for driving the high voltage generation apparatus.

Another electric potential therapy apparatus comprises an electric potential treatment device provided with a main electrode and an opposed electrode, a high voltage generation apparatus for applying a high voltage to the respective electrodes, induced current control means for causing an extremely small amount of induced current to flow in respective areas composing a human body trunk by controlling the applied voltage applied to the respective electrodes, and a power source for driving the high voltage generation apparatus.

Still another electric potential therapy apparatus comprises an electric potential treatment device provided with a main electrode and an opposed electrode, a high voltage generation apparatus for applying a high voltage to the respective electrodes, induced current control means for causing an extremely small amount of induced current to flow in respective areas composing a human body trunk by controlling the distance between the opposed electrode and the human body trunk surface, and a power source for driving the high voltage generation apparatus.

A preferred form of any of the electric potential therapy apparatus described above is characterized in that the high voltage generation apparatus is provided with a configuration made by grounding the middle point of a booster coil.

Another preferred form of any of the electric potential therapy apparatus described above is characterized in that an intensity E of a body surface electric field at respective areas of a human body is obtained by an expression of $E = I/\varepsilon_o \omega S$.

A further preferred form of any of the electric potential therapy apparatus described above is characterized in that the induced current in respective areas of a human body is obtained by measuring the current flowing in the section of a measured area and converting it into a voltage signal, converting the voltage signal into an optical signal, and thereafter, reconverting the optical signal into a voltage signal, and analyzing the waveform and frequency.

Still another preferred form of the electric potential therapy apparatus described above is characterized in that the applied voltage and the induced current of respective areas composing a human body trunk are in proportional relation.

Further preferred form of the electric potential therapy apparatus described above is characterized in that the applied voltage is made by adjusting the induced current density of respective areas obtained from the induced current flowing in respective areas composing a human body trunk to about 10.0 mA/m$^2$ or less.

Still another preferred form of the electric potential therapy apparatus described above is characterized in that the opposed electrode is placed at a position on the head, or at any position of head, both shoulders, abdomen, waist and hips of a human body, and the distance with the human body trunk surface is respectively about 1 to 25 cm.

In still another modification of the electric potential therapy apparatus described above, the distance between the human body trunk surface and the opposed electrode is characterized by that the induced current density flowing in respective areas composing a human body trunk is adjusted to about 10.0 mA/m$^2$ or less.

A further preferred form of the electric potential therapy apparatus described above is characterized in that the opposed electrode is the ceiling, wall, floor, furniture or others.

A control method of an optimal dose amount for a human body area comprises the steps of applying a high voltage to an electrode, controlling the dose amount obtained by the product of an induced current value flowing in areas composing a human body trunk and an induced current flowing time, and supplying the dose amount to respective areas of a human body.

Another control method of an optimal dose amount for a human body area comprises the steps of applying a high voltage to an electrode, controlling the dose amount obtained by the product of an applied voltage applied to the main electrode and the opposed electrode and an applying time, and supplying the dose amount to respective areas of a human body.

A modification of the optimal dose amount control method for a human body area described above is characterized in that a dose amount effective for lumbago is obtained by the product of an induced current value flowing in the respective areas of a human body trunk about 10.0 mA/m$^2$, preferably about 0.5 mA/m$^2$ to about 5.0 mA/m$^2$ and the current supply time about 30 min.

Another modification of the optimal dose amount control method for a human body area described above is characterized in that a dose amount effective for lumbago is obtained by the product of an applied voltage about 10 to 20 KV, preferably 15 KV and the current supply time about 30 min.

According to the electric potential therapy apparatus and human body area optimal dose amount control method of the present invention, a fine area electric field therapy can be performed effectively for each individual, by controlling so as to supply an optimal and effective induced current to the respective areas of a human body trunk of the individual, and a high safety can also be secured for the respective areas of a human body trunk. Moreover, many subjects recognize virtue and effect especially for lumbago by experiment.

BRIEF DESCRIPTION OF THE DRAWINGS

Fig. 1 is a schematic view of an electric potential therapy apparatus of the present invention;
Fig. 2 is a diagram showing an electric configuration of the electric potential therapy apparatus of the present invention;
Figs. 3(a) to 3(c) are a photographed front view of a virtual human body, a perspective view thereof and a view showing the state wherein an electric field measurement sensor is attached to a neck portion thereof, respectively;
Fig. 4 is a diagram showing a measurement apparatus for measuring the induced current of the electric potential therapy apparatus of the present invention;
Fig. 5 is a graph showing the relation between an applied voltage and the induced current;
Fig. 6 is a graph showing the relation between a head electrode position and a neck induced current;
Figs. 7(a) and 7(b) are views showing an electric potential therapy apparatus of another embodiment of the present invention;
Figs. 8(a) and 8(b) are views showing an electric potential therapy of a still another embodiment of the present invention; and
Figs. 9(a) and 9(b) are views showing an electric potential therapy apparatus of still another embodiments of the present invention;

DESCRIPTION OF THE PREFERRED EMBODIMENTS

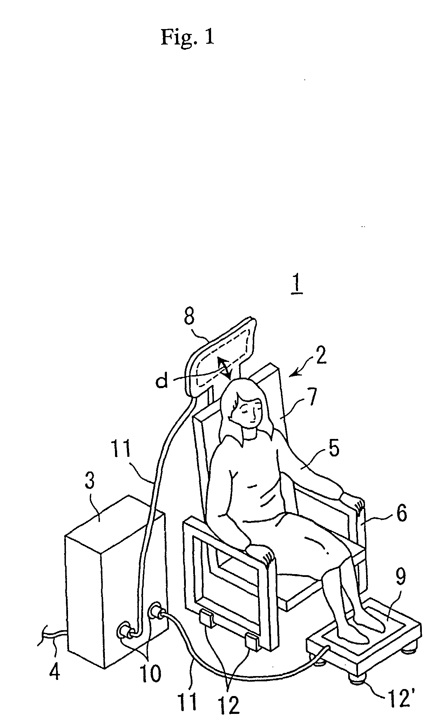

Fig. 1 is a schematic view of an high voltage generation apparatus (1) showing an embodiment of the present invention. Namely, the electric potential therapy apparatus (1) comprises an electric potential treatment device (2), a high voltage generation apparatus (3) and a commercial power source (4). The electric potential treatment device (2) comprises a chair (7) with armrests (6) where a subject (5) sits, a first electrode (8) as opposed electrode attached to the chair upper end and arranged above a head top of the subject (5), and a second electrode (9) as ottoman electrode which is a main electrode where the subject (5) puts his/her legs on the top face thereof. Note that this first electrode (8), as opposed electrode of the second electrode (9) which is a main electrode, may otherwise be ceiling, wall, floor, furniture or others. The high voltage generation apparatus (3) generates a high voltage to impress a voltage to the first electrode (8) and second electrode (9). The high voltage generation apparatus (3) is generally installed under the chair (7), among four legs and on the floor, or in the vicinity of the chair (7). A distance (d) [described below] between the first electrode (8) and the head top can be varied.

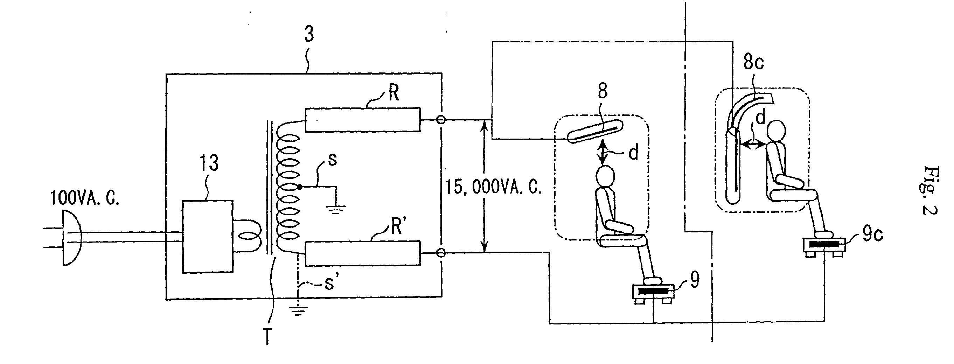

The first electrode (8) and the second electrode (9) comprise a composition to be surrounded by an insulation material. This second electrode (9) is connected to a high voltage output terminal (10) of the high voltage generation apparatus (3) by an electric cord (11). It is also provided with the high voltage output terminal (10) to impress a voltage to the first electrode (8) and the second electrode (9). In addition, the chair (7) and the second electrode (9) comprise insulators (12), (12)' at the contact positions with the floor. The high voltage generation apparatus (3) has, as described below for an electric configuration block diagram in Fig. 2, a booster transformer (t) for boosting a voltage of the commercial power source 100V AC to, for example, 15,000 V, and current limitation resistors (R), (R)' for controlling the current flowing to the respective electrodes. This high voltage generation apparatus (3) has a configuration wherein a middle point (s) of a booster coil (T) is grounded, and the ground voltage is set to the half of the boosted voltage. Besides, as shown by the illustrated provisory line, a point (s') can be grounded. Here, as the block diagram shown in Fig. 2, a high voltage whose high voltage side middle point (s) is grounded by the booster transformer (T) is obtained from an 100V AC power source passing through a voltage controller (13) of the high voltage generation apparatus (3) and further, respective high voltages are connected to the first electrodes (8), (8c) or the like (mentioned below) and the second electrodes (9), (9c) or the like (mentioned below) through the current limitation resistors (R), (R') for human body protection. And, the electric potential therapy apparatus (1) is provided with induced current control means. This induced current control means can cause an extremely small amount of induced current to flow in respective areas composing a human body trunk of the subject (5) with control of the body trunk electric field by varying the applied voltage to be applied to the first electrode (8) and second electrode (9), and a distance (d) between the first electrode (8) and the human body trunk surface, or by controlling the applied voltage to be applied to the first electrode (8) and second electrode (9), or further by varying the distance (d) between the first electrode (8) and the human body trunk surface.

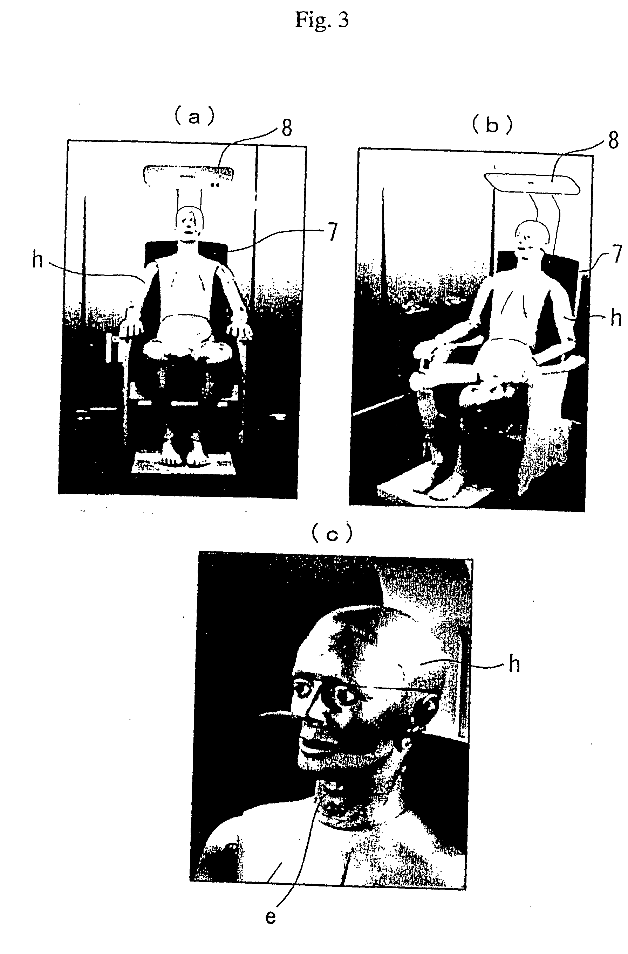

As mentioned above, measurement data of the human area electric field and the induced current shall be obtained using a virtual human body (h)[simulated human body model] as human body model, as shown by photographed pictures of Figs. 3(a), 3(b) and 3(c). This virtual human body (h) is made of PVC and the surface thereof is coated with a mixed solution of silver and silver chloride. This is to make the resistance (1K $\Omega$ or less) thereof similar to the resistance of a real human body. And this virtual human body (h) is actually used largely worldwide as nursing simulator, made to represent the dimensions of an average human body and is 174 cm tall. The dimensions of the surrounding of respective areas of the virtual human body (h) and their section are to be described in Table 1. Such virtual human body (h) is used, because it is necessary to implant a measuring instrument in the human body in order to section a real human body and measure the induced current of respective portions, and further it is difficult to measure suppressing human body minute movement. On the other hand, it is largely possible to apply data obtained by the virtual human body (h) to a real human body.

[Table 1]

Table 1 :

| Measurement of Virtual Human Body's Area | | |
|---|---|---|
| Section of Area | Circumference (mm) | Cross Sectional Area (m$^2$) |
| Eye | 550 | 0.02407 |
| Nose | 475 | 0.01795 |

Table 1 : (continued)

| Measurement of Virtual Human Body's Area | | |
|---|---|---|
| Section of Area | Circumference (mm) | Cross Sectional Area ($m^2$) |
| Neck | 328 | 0.00856 |
| Chest | 770 | 0.04718 |
| Pit of the stomach | 710 | 0.04012 |
| Arm | 242 | 0.00466 |
| Wrist | 170 | 0.00230 |
| Trunk | 660 | 0.03466 |
| Thigh | 450 | 0.01611 |
| Knee | 309 | 0.00760 |
| Ankle | 205 | 0.00334 |

The virtual human body (h) for measurement installed in the electric potential treatment device (2) shown in Fig. 1 is as shown in Fig. 3(a) and 3(b). Fig. 3(a) is a front view of the virtual human body (h) and Fig. 3(b) is a perspective view thereof.

Now, it will be described that the induced current control means mentioned above can cause an extremely small amount of induced current to flow in respective areas composing a human body trunk with control of the body trunk electric field by varying the applied voltage to be applied to the first electrode (1) and second electrode (2), and the distance (d) between the first electrode (8) and the human body trunk surface. The body surface electric field is measured by attaching a disk shaped electric field measurement sensor (e) to a measurement area of the virtual human body (h) shown in Fig. 1. Note that Fig. 3(c) is a view showing the state wherein the electric field measurement sensor (e) is attached to a neck portion of the virtual human body (h). Respective areas are measured under the condition of 115 V/60 Hz and 120 V/60 Hz.

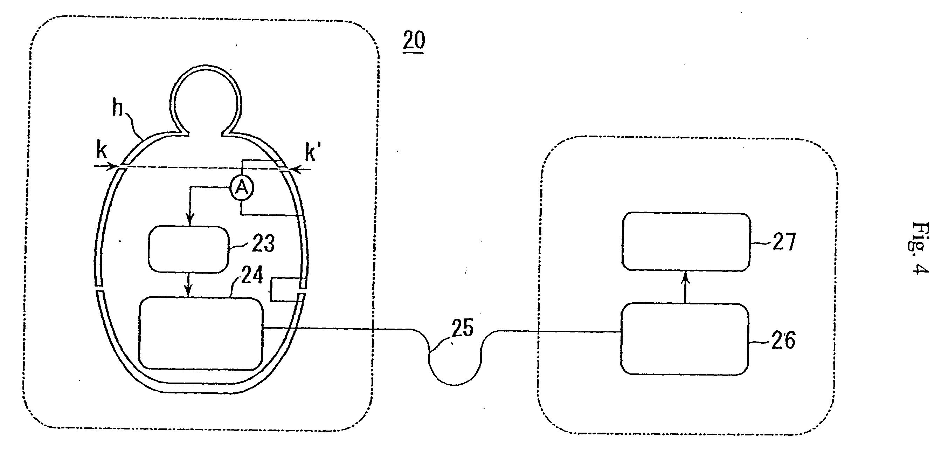

On the other hand, a measurement method of an induced current and a measurement apparatus therefor are shown in Fig. 4. In the induced current measurement apparatus (20), as shown in Figs. 3(a) and 3(b), the virtual human body (h) is put on the chair (7) in a normal sitting state. The first electrode (8) over the head, which is the opposed head, is adjusted and installed to be 11 cm from above a head of the virtual human body (h). The measuring method is achieved by measuring respective portions such as, for example, the illustrated k-k' line portion, transferring the induced current waveform through optical transfer, and observing this waveform at the ground side of the induced current measurement apparatus (20). Here, the applied voltage is 15,000 V. In this measuring method, the measurement of the current induced at the section of respective areas of the virtual human body (h) obtains the induced current by creating a short-circuit (22) [not shown] of a current flowing across the section of the virtual human body (h) using two lead wires. The measured induction current is converted into a voltage signal through an I/V converter (23). Next, this voltage signal is converted into an optical signal by an optical analog data link at the transmission side.

These optical signals are transferred to an optical analog data link (26) at the reception side, through an optical fiber cable (25) and converted into a voltage signal. This voltage signal is then processed by a frequency analyzer (27) for frequency analysis by a waveform observation and analysis recorder. A buffer and an adder are disposed between the I/V converter (23) and the optical analog data link (24) at the transmission side [not shown]. Thus, electric field value and induction current measured at the 115 V/60 Hz and 120 V/60 Hz, at the position of respective areas of the virtual human body (h), are shown in Table 2. If the electric field value is different from this Table 2, accordingly, it is known that the induced current value flowing there is also different. Therefore, it is supposed that it is evident that the induced current effective for respective areas of a real human body trunk can be obtained by changing the electric field of the concerned respective areas.

[Table 2]

Table 2 :

| Relationship between Electric Field Value and Induced Current Value | | | | |
|---|---|---|---|---|
| Section of Area | @115V/50Hz | | @120V/60Hz | |
| | Electric Field Value (kV/m) | Induced Current (μA) | Electric Field Value (kV/m) | Induced Current (μA) |
| Top of the head | 182 | 0.72 | 190 | 0.90 |
| Front of the head | 81 | 0.32 | 84 | 0.40 |
| Back of the head | 113 | 0.44 | 118 | 0.55 |
| Side of the neck | 16 | 0.06 | 16 | 0.08 |
| Shoulder | 37 | 0.15 | 38 | 0.18 |
| Chest | 19 | 0.08 | 20 | 0.10 |
| Arm | 29 | 0.11 | 30 | 0.14 |
| Elbow | 33 | 0.14 | 34 | 0.17 |
| Back | 52 | 0.20 | 54 | 0.25 |
| Back of the hand | 21 | 0.08 | 22 | 0.10 |
| Coccyx | 42 | 0.17 | 43 | 0.21 |
| Knee | 11 | 0.05 | 12 | 0.06 |
| Patella | 21 | 0.08 | 22 | 0.10 |
| Tip of the foot | 3.4 | 0.01 | 3.5 | 0.02 |
| Bottom of the foot | 348 | 1.37 | 363 | 1.72 |

Besides, the body surface electric field E can be obtained by using the following equation, from the induced current value of the respective areas obtained by the measurement method of the induced current of respective areas shown in Fig. 4. Namely, $E = I/\varepsilon_o\omega S$. Here, $\omega$ is $2\pi f$ (f; frequency), S is a section of the electric field measurement sensor, $\varepsilon_o$ is an induction rate in the vacuum, and I is an induced current.

When the induced current of respective areas is obtained by the aforementioned method, an induced current density J of respective areas can be obtained using the following expressions. Namely, $A = 2\pi r$, $B = \pi r^2$, $B = A^2/4\pi$, $J = I/B$, where A is a circumference, B is a circle area, r is a radius, I is a measured current, and J is an induced current density.

Next, it will be described that the induced current control means mentioned above can cause an extremely small amount of induced current to flow in respective areas of a human body trunk, when the electric potential therapy is performed, by controlling the voltage of the first electrode (8) and the applied voltage applied to the second electrode (9).

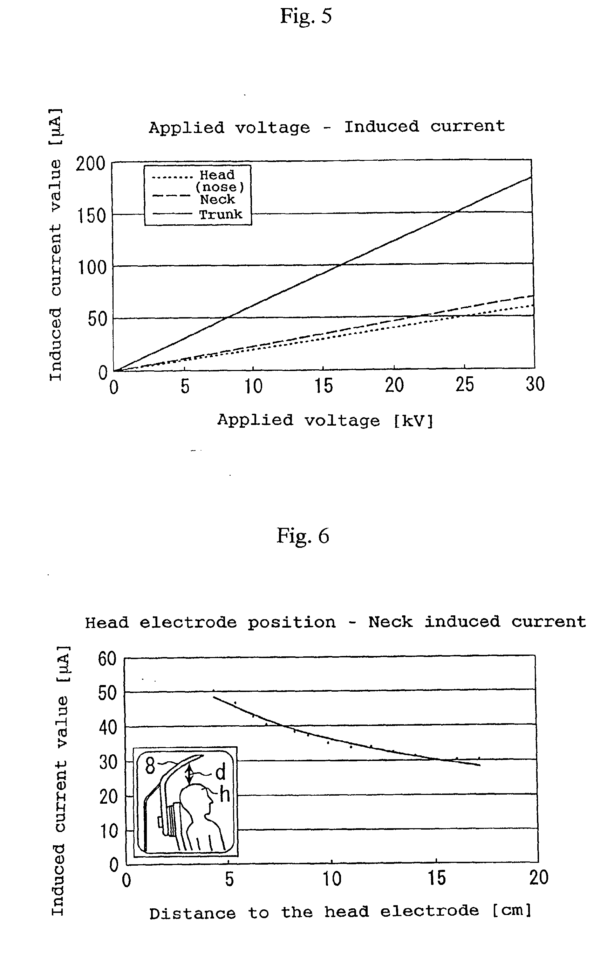

Table 3 shows the induced current (μA) and the induced current density (mA/m²) to the applying current (KV) at the head (nose), neck and trunk under 120 V/60 Hz. Fig. 5 shows the relation between the respective applying currents (KV) and the induced current (μA) at the head (nose), neck and trunk based on the results of the aforementioned Table 3. It is understood that the applying current and the induced current in respective areas are in proportional relationship from this Fig. 5. The induced current density J of respective areas can by obtained from the induced current value using the aforementioned expression. And here, the applied voltage is required to control the induced current density of respective areas to about 10.0 mA/m² or less. This value of about 10.0 mA/m² or less is a value equal or inferior to the safety standard defined by the International Commission on Non Ionizing Radiation Protection. It is also understood that the applying current at the head, neck and trunk is stronger in the order of trunk, neck and head (nose) under the same applied voltage.

[Table 3]

Table 3:

| Applied Voltage and Induced Current | | | | | | |
|---|---|---|---|---|---|---|
| Applied voltage [kV] | Induced current Value | | | Induced Current Density (mA/m$^2$) | | |
| | Head Portion (nose) | Neck Portion | Trunk Portion | Head Portion (nose) | Neck Portion | Trunk Portion |
| 0 | 0 | 0 | 0 | 0.0 | 0.0 | 0.0 |
| 5 | 10 | 11 | 30 | 0.6 | 1.3 | 0.9 |
| 10 | 20 | 23 | 61 | 1.1 | 2.6 | 1.7 |
| 15 | 30 | 34 | 91 | 1.7 | 3.9 | 2.6 |
| 20 | 40 | 45 | 121 | 2.2 | 5.2 | 3.5 |
| 25 | 50 | 57 | 152 | 2.8 | 6.6 | 4.4 |
| 30 | 60 | 68 | 182 | 3.3 | 7.9 | 5.2 |

Next, it will be described that the induced current control means can cause an extremely small amount of induced current to flow in respective areas of a human body trunk, by making the distance (d) between the first electrode (8) and the human body surface variable. Table 4 shows the variation of an induced current value flowing in the neck portion of a human body and an induced current density, by changing the distance (d) between the first electrode (8) of the head portion and the human body head top portion. Fig. 6 shows the relation between the distance (d) to the first electrode (8) at the head portion of the virtual human body (h) and the neck portion induced current.

[Table 4]

Table 4:

| Change in Induced Current according to the Location of the Head Portion Electrode | | |
|---|---|---|
| Head Portion Electrode - Top Portion of the Head Distance | Induced Current Value | Induced Current Density |
| (cm) | (μA) | (mA/m$^2$) |
| 4.3 | 50 | 5.8 |
| 5.4 | 46 | 5.4 |
| 6.3 | 43 | 5.0 |
| 6.9 | 40 | 4.7 |
| 8.3 | 39 | 4.5 |
| 9 | 38 | 4.4 |
| 9.9 | 35 | 4.1 |
| 11 | 34 | 3.9 |
| 12 | 34 | 3.9 |
| 13 | 33 | 3.8 |
| 14 | 31 | 3.7 |
| 15 | 30 | 3.5 |

Table 4:  (continued)

| Change in Induced Current according to the Location of the Head Portion Electrode | | |
|---|---|---|
| Head Portion Electrode - Top Portion of the Head | Induced Current Value | Induced Current Density |
| Distance | | |
| (cm) | ($\mu$A) | (mA/m$^2$) |
| 16.1 | 30 | 3.5 |
| 17.2 | 30 | 3.5 |

As it is understood from this Table 4, the induced current becomes approximately stable at the distance; 15 cm or more, and at 30 $\mu$A. Therefore, the induced current value is approximately free from the influence of the head portion first electrode (8). Thus, the induced current value can be controlled by varying the distance (d) within 15 cm. It is desirable that the induced current density of the neck portion, a part of the trunk of the virtual human body (h) obtained from this induced current value is controlled to about 10.0 mA/m$^2$ or less, preferably to about 3.0 mA/m$^2$ to about 6.0 mA/m$^2$.

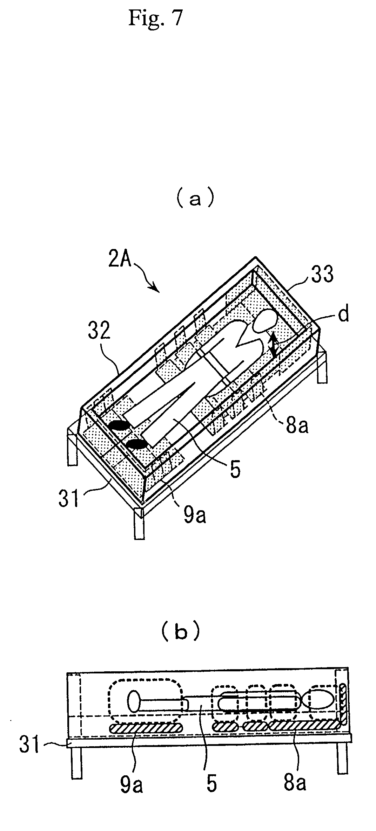

An electric potential treatment device (2A) provided with another structure is shown in Fig. 7(a) [perspective view] and Fig. 7(b) [side view]. This electric potential treatment device (2A) has a bed type. A box (32) for containing the subject (5) is disposed on a bed base (31). Respective electrodes are provided in this box (32). In short, it is provided with a first electrode (8a) as opposed electrode and a second electrode (9a) placed at a leg portion of the human body as main electrode. The first electrode (8a) is placed at head, both shoulders, abdomen, legs and hips of a human body or other areas. And preferably, the first electrode (8a) has the shape, breadth and area approximately equal to head, both shoulders, abdomen and hips of a human body.

Besides, blank areas in these drawings show the points where no electrodes are disposed. Electrodes are disposed in an insulator (33). A sofa made of an insulator (not shown) is put on the respective electrodes on the bed base (31). There, sofas of different thickness are prepared. The distance (d) between the human body surface and the first electrode (8a) [refer to Fig. 1 and Fig. 2 mentioned above] can be changed easily by putting sofas of thus different thickness on the bed base (31). In such electric potential treatment device (2A), as the induced current control means as mentioned above, an extremely small amount of induced current can be made to flow in the respective areas of a human body trunk with control of the body surface electric field by making the applied voltage to be applied to the first electrode (8a) and second electrode (9a), and the distance (d) between the first electrode (8a) and the human body trunk surface variable, or by controlling the applied voltage to be applied to the first electrode (8a) and second electrode (9a), or by changing the distance (d) between the first electrode (8a) and the human body trunk surface.

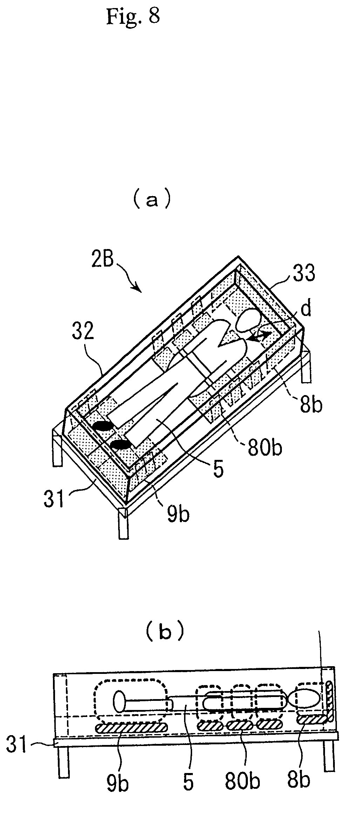

An electric potential treatment device (2B) provided with still another structure is shown in Fig. 8(a) [perspective view] and Fig. 8(b) [side view]. This electric potential treatment device (2B) is also bed type, and a box (32) for containing the subject (5) is disposed on a bed base (31). Respective electrodes are provided in this box (32). Here, it is provided with a first electrode (8b) as opposed electrode disposed at a head portion, a second electrode (9b) is placed at a leg portion of the human body as main electrode, and another first electrode (80b) as opposed electrode disposed at a waist upper body portion. The first electrode (8b) is placed at the human body head, and preferably, the first electrode (8a) has the shape, breadth and area approximately equal to the human body head. In addition, the second electrode (9b) placed at a leg portion of the human body as mentioned before. Moreover, the another first electrode (80b) has the shape, breadth and area approximately equal to the human body head, both shoulders, abdomen and hips. These electrodes are arranged in an insulator (33). In this electric potential treatment device (2B) also, similarly as Fig. 7, the distance (d) between the human body surface and the first electrode (8b) or another electrode (80b) can be changed easily by putting sofas of different thickness on the bed base (31) at the position corresponding at least to the first electrode (8b) and another electrode (80b). Besides, blank areas in these drawings show the points where no electrode is disposed. In such electric potential treatment device (2B), as mentioned above, the induced current control means can respectively control the body surface electric field and cause an extremely small amount of induced current to flow in the respective areas of a human body trunk by making the applied voltage to be applied to the first electrode (8b) and another first electrode (80b), and the distance (d) between the first electrode (8b), another first electrode (80b) and the human body trunk surface variable, or by controlling the applied voltage to be applied to the first electrode (8b), second electrode (9b) and another first electrode (80b), or by changing the distance (d) between the first electrode (8b), another first electrode (80b) and the human body trunk surface.

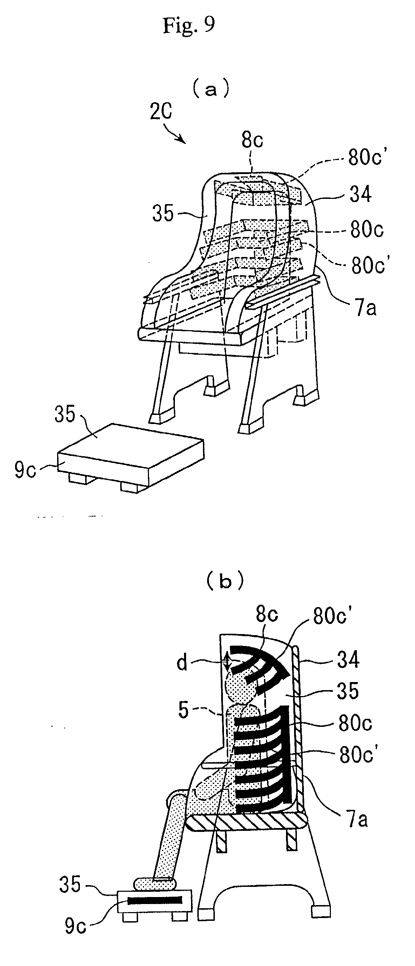

An electric potential treatment device (2C) provided with still another structure has a chair type shown in Fig. 9(a) [perspective view] and Fig. 9(b) [side view illustrating the positional relationship between the subject (5) and respective electrodes painted in black]. The chair (7a) is provided with a front open cover body (34) covering the subject (5). This cover body (34) is provided with a first electrode (8c) as opposed electrode to receive the head of the subject (5), a second electrode (9c) which is an ottoman electrode as main electrode, and another first electrode (80c) disposed at the position of shoulder to waist of the sitting posture as opposed electrode disposed at the waist upper body portion. The another first electrode (80c) has a plurality of side electrodes (80c') so as to cover respectively the body of the subject (5) from the side. Preferably, the first electrode (8c) is arranged along the human body head portion, and another first electrode (80c) is disposed in a plurality of stages along the longitudinal direction from both shoulders to the waist. These first electrode (8c), another first electrode (80c), the side electrodes (80c') and second electrode (9c) are arranged in an insulating material (35).

A cushion member made of insulator is detachably attached to the cover body (34). Thus, the attachment of a cushion member different in thickness can vary the distance between the human body surface and the first electrodes (8c), (80c), (80c'). In such electric potential treatment device (2c) also, as mentioned above, the induced current control means can respectively control the body surface electric field and flow an extremely small amount of induced in the respective areas of a human body trunk by making the applied voltage to be applied to the first electrodes (8c), (80c), (80c') as opposed electrode, and the second electrode (9c), and the distance (d) between the first electrode (8c), (80c), (80c') and the human body trunk surface variable, or by controlling the applied voltage to be applied to the first electrode (8c), (80c), (80c') and second electrode (9c) and further, by changing the distance (d) between the first electrode (8c), (80c), (80c') and the human body surface.

In Fig. 1 mentioned above, the distance (d) between the first electrode (8) above the head and the human body trunk surface of the subject (5) is set to about 1 to 25 cm, in Fig. 7(a) and Fig. 8(a), the distance (d) between the first electrode (8a), (8b) and the human body trunk surface of the subject (5) to about 1 to 25 cm, preferably about 3 to 25 cm, and in Fig. 9(a), the distance (d) between the first electrode (8c), (80c), (80c') and the subject (5) human body trunk surface is set to about 1 to 25 cm, preferably about 4 to 25 cm.

According to the electric potential therapy apparatus (1) of the present invention, a higher therapeutic effect can be obtained, even for the same period of time equal to that in the conventional method, by increasing the induced current amount even in a state where a high voltage is applied. In addition, the treatment can be completed within a time shorter than before. And further, to obtain the same therapeutic effect, an induced current of the same value as the prior art can be obtained with a lower voltage and in a same treatment time as before.

The electric potential therapy apparatus (1) of the present invention is designed to be exempt, as much as possible, from high output electronic noise, high level radio frequency noise and strong magnetic field. In order to reduce the influence of electromagnetic field interference with the electric potential therapy apparatus (1), it is preferable to use driven mechanical switch, relay and electric motor or electric timer or other electric components rather than electronic components, semiconductor, power component (such as thyristor, triac) electronic timer or EMI sensible microcomputer for the designing and manufacturing thereof. However, as electronic functional component, the electronic serial bus switching regulator for optical emitter diode power source is effective, and this optical emitter diode is used as an optical source for informing the subject or the operator of the active or inactive state of the electric potential therapy apparatus of the present invention.

Now, a fact found in a therapeutic experiment to 300 or more cases of lumbago will be described below and it was found that it is effective to the treatment of human body lumbago and the optimal dose taking account of human body safety is controlled and obtained as follows. In short, the optimal dose amount is obtained by controlling the product of the induced current value flowing in areas composing a human body trunk and the induced current flowing time. Otherwise, it is obtained by controlling the product of the applied voltage sum of the first electrode voltage and the second electrode voltage, and the applying time thereof. Here, Table 5 shows the induced current value measured with 115 V/50 Hz at the section of respective areas composing the trunk of the virtual human body (h), and the induced current density obtained by calculation from this induced current value, taking the dimensions of the virtual human body (h) of the Table 1 into consideration. From Table 5, measured values of induced current ($\mu$A) in respective areas composing the trunk of human body and the calculated values of induced current density (mA/m$^2$) are as follows: Eye; 18/0.8, nose; 24/1.3, neck; 27/3.1, chest; 44/0.9, pit of the stomach; 8.6/1.6, and trunk; 91/2.8.

[Table 5]

Table 5:

| Area, Induced Current Value, and Induced Current Density | | |
|---|---|---|
| Section of Area | Induced Current @115V/50Hz ($\mu$A) | Induced Current Density @115V/50Hz (mA/m$^2$) |
| Eye | 18 | 0.8 |

Table 5:   (continued)

| Area, Induced Current Value, and Induced Current Density | | |
|---|---|---|
| Section of Area | Induced Current @115V/50Hz (μA) | Induced Current Density @115V/50Hz (mA/m$^2$) |
| Nose | 24 | 1.3 |
| Neck | 27 | 3.1 |
| Chest | 44 | 0.9 |
| Pit of the stomach | 65 | 1.6 |
| Arm | 8.6 | 1.8 |
| Wrist | 3.1 | 1.3 |
| Trunk | 73 | 2.1 |
| Thigh | 46 | 2.8 |
| Knee | 52 | 6.8 |
| Ankle | 58 | 17 |

Moreover, based on the aforementioned induced current and induced current density, the induced current and induced current density at 120 V/60 Hz are calculated according to the following expression 1 and expression 2.

[Expression 1]]

Induced Current;

$$I(60Hz)=I(50Hz)\times 60/50\times 120/115$$

[Expression 2]

Induced Current Density;

$$J(60Hz)=J(50Hz) \times 60/50\times 120/115$$

Table 6 shows the calculation result of the induced current and induced current density of respective areas that are human body trunk at 120 V/60 Hz. From Table 6, measured values of induced current (μA) in respective areas composing the trunk of human body and the calculated value of induced current density (mA/m$^2$) are as follows: Eye; 23/0.9, nose; 30/1.7, neck; 34/3.9, chest; 55/1.2, pit of the stomach; 11/2.3, and trunk; 114/3.6.

[Table 6]

Table 6:

| Area, Induced Current Value, and Induced Current Density | | |
|---|---|---|
| Section of Area | Induced Current @125V/60Hz (μA) | Induced Current Density @120V/60Hz (mA/m$^2$) |
| Eye | 23 | 0.9 |
| Nose | 30 | 1.7 |
| Neck | 34 | 3.9 |
| Chest | 55 | 1.2 |

Table 6:   (continued)

| Area, Induced Current Value, and Induced Current Density | | |
|---|---|---|
| Section of Area | Induced Current @125V/60Hz (μA) | Induced Current Density @120V/60Hz (mA/m$^2$) |
| Pit of the stomach | 81 | 2.0 |
| Arm | 11 | 2.3 |
| Wrist | 3.9 | 1.7 |
| Trunk | 91 | 2.6 |
| Thigh | 57 | 3.6 |
| Knee | 64 | 8.5 |
| Ankle | 72 | 22 |

When the distance between the electrode and the human body area is fixed, the voltage applied as mentioned above, and the induced current flowing in the body trunk respective areas of a human body are in proportional relationship. Therefore, when a human body is treated with a chair, the optimal dose amount can be obtained by controlling the product of the applied voltage and the applying time, because the electric field intensity of respective areas of a human body is almost decided by the applied voltage, if the distance between the electrode and the human body is decided in a manner of the greatest common divisor. In case of using the electric potential therapy apparatus of the present invention, the product of voltage and time is preferably 450 KV/min. In short, it was found that the therapeutic effect can be enhanced at the voltage; about 10 KV to about 20 KV, preferably about 15 KV, applying time; about 30 min.

**Claims**

1. An electric potential therapy apparatus, comprising:

   an electric potential treatment device provided with a main electrode and an opposed electrode;
   a high voltage generation apparatus for applying a high voltage to the respective electrodes;
   induced current control means for causing an extremely small amount of induced current to flow in respective areas composing a human body trunk with control of an electric field of the body trunk, by varying the applied voltage to be applied to the respective electrodes and the distance between the opposed electrode and the human body trunk surface; and
   a power source for driving the high voltage generation apparatus.

2. An electric potential therapy apparatus, comprising:

   an electric potential treatment device provided with a main electrode and an opposed electrode;
   a high voltage generation apparatus for applying a high voltage to the respective electrodes;
   induced current control means for causing an extremely small amount of induced current to flow in respective areas composing a human body trunk by controlling the applied voltage applied to the respective electrodes; and
   a power source for driving the high voltage generation apparatus.

3. An electric potential therapy apparatus, comprising:

   an electric potential treatment device provided with a main electrode and an opposed electrode;
   a high voltage generation apparatus for applying a high voltage to the respective electrodes;
   induced current control means for causing an extremely small amount of induced current to flow in respective areas composing a human body trunk, by controlling the distance between the opposed electrode and the human body trunk surface; and
   a power source for driving the high voltage generation apparatus.

4. The electric potential therapy apparatus according to any one of claims 1 to 3, wherein the high voltage generation apparatus is provided with a configuration made by grounding the middle point of a booster coil.

5. The electric potential therapy apparatus according to any one of claims 1 to 3, wherein an intensity E of a body surface electric field at respective areas of a human body is obtained by an expression of $E = I/\varepsilon_o \omega S$.

6. The electric potential therapy apparatus of any one of claims 1 to 3, wherein the induced current in respective areas of a human body is obtained by measuring the current flowing in the section of a measured area and converting it into a voltage signal, converting the voltage signal into an optical signal, and thereafter, reconverting the optical signal into a voltage signal, and analyzing the waveform and frequency.

7. The electric potential therapy apparatus according to claim 1 or 2, wherein the applied voltage and the induced current of respective areas composing a human body trunk are in proportional relation.

8. The electric potential therapy apparatus according to claim 1 or 2, wherein the applied voltage is made by adjusting the induced current density of respective areas obtained from the induced current flowing in respective areas composing a human body trunk to about 10.0 mA/m$^2$ or less.

9. The electric potential therapy apparatus according to claim 1 to 3, wherein the opposed electrode is placed at a position on the head, or at any position of head, both shoulders, abdomen, waist and hips of a human body, and the distance with the human body trunk surface is respectively about 1 to 25 cm.

10. The electric potential therapy apparatus according to claims 1 or 3, wherein the distance between the human body trunk surface and the opposed electrode is **characterized by** that the induced current density flowing in respective areas composing a human body trunk is adjusted to about 10.0 mA/m$^2$ or less.

11. The electric potential therapy apparatus according to claim 1 or 3, wherein the opposed electrode is the ceiling, wall, floor, furniture or others.

12. A control method of an optimal dose amount for a human body area, comprising the steps of:

    applying a high voltage to an electrode;
    controlling a dose amount obtained by a product of an induced current value flowing in areas composing a human body trunk and an induced current flowing time; and
    supplying the dose amount to respective areas of a human body.

13. A control method of an optimal dose amount for a human body area, comprising the steps of:

    applying a high voltage to an electrode;
    controlling a dose amount obtained by a product of an applied voltage applied to a main electrode and an opposed electrode and an applying time; and
    supplying the dose amount to respective areas of a human body.

14. The optimal dose amount control method for a human body area according to claim 12, wherein a dose amount effective for lumbago is obtained by the product of an induced current value flowing in the respective areas of a human body trunk of about 10.0 mA/m$^2$m, preferably about 0.5 mA/m$^2$ to about 5.0 mA/m$^2$ and the current supply time of about 30 min.

15. The optimal dose amount control method for a human body area according to claim 13, wherein a dose amount effective for lumbago is obtained by the product of an applied voltage about 10 to 20 KV, preferably 15 KV and the current supply time about 30 min.

Fig. 1

## Fig. 2

Fig. 3

( a )

( b )

( c )

Fig. 4

Fig. 5

Applied voltage - Induced current

Fig. 6

Head electrode position - Neck induced current

Fig. 7

（ a ）

（ b ）

Fig. 8

（a）

（b）

Fig. 9

(a)

(b)